# EUROPEAN PATENT SPECIFICATION

(11) **EP 4 431 027 B1**
(45) Date of publication and mention of the grant of the patent: **12.03.2025**
(21) Application number: 23215590.3
(22) Date of filing: 11.12.2023
(51) Int. Cl.: A61B 10/02, A61B 10/04

(54) **INSTRUMENT FOR TAKING A TISSUE SAMPLE**
GERÄT ZUR ENTNAHME VON GEWEBEPROBEN
DISPOSITIF DE PRELEVEMENT D'UN ECHANTILLON DE TISSU

(30) Priority: 13.03.2023 BE 202305182
(43) Date of publication of application: 18.09.2024
(73) Proprietor: Janssens, Jacques Philibert, 3512 Stevoort (BE)
(72) Inventor: Janssens, Jacques Philibert, 3512 Stevoort (BE)
(74) Representative: Bureau M.F.J. Bockstael NV

(56) References cited:
- EP-B1- 2 623 036
- BE-A1- 1 028 018
- US-A1- 2012 197 157
- US-A1- 2020 015 852

## Description

The present invention relates to an instrument for taking a tissue sample.

More specifically it relates to an instrument of the type described in WO 02/065919, or in BE 1028018 A1, in which a spiral or helical tissue receiving element is turned in the tissue from which a sample must be taken, after which the tissue around this tissue receiving element is cut with a sharp cannula around the tissue receiving element and is torn off at the distal end of the tissue receiving element, thereby obtaining a tissue sample in the tissue receiving element.

Such instruments have a greater tissue sample volume / needle diameter ratio than a so-called tru-cut biopsy needle.

EP 2623036 B1 discloses the features of the preamble of claim 1 and is an improvement of the aforementioned instrument, whereby the diameter of the outer surface of the spiral or helical tissue receiving element decreases toward the distal end of the tissue receiving element.

Consequently a space is created between the cutting cannula and the tissue receiving element when the cannula is slid over the tissue receiving element.

This has the advantage that when the spiral or helical tissue receiving element were to expand when being turned into harder tissues, the desired movement of the cutting cannula is not disrupted.

In addition, the aforementioned space also offers room for the so-called lateral connective tissue fibres that run through the tissue of organs and extend partly transversely to the longitudinal direction of the tissue receiving element and that are not cut on the outside of the helix. Consequently, the tissue sample is held between the helix bodies such that the distal tearing will not cause tissue loss.

Most such instruments for taking a tissue sample have a small diameter.

For endoscopic applications a scope with a working channel is used in which the instrument for taking a tissue sample is inserted.

The diameter of the working channel is linked to the distance over which the endoscope can be inserted in the patient. Thus, the endoscope can be inserted maximally until the diameter of the organ, for example the airway, becomes smaller than the diameter of the scope.

The most common endoscopes have a working channel with a diameter that can only amount to 1.8 millimetres.

The result is that the diameter of the outer surface of the spiral or helical tissue receiving element can maximally only amount to less than 3 millimetres.

The result is that the tissue sample is very small.

Preclinical tests have shown that the dimensions of the tissue sample decrease overproportionally as the diameter of the tissue receiving element gets smaller.

However, in order to obtain a good and representative tissue sample, care must be taken to collect as much tissue as possible.

US 2012/0197157 A1 discloses an instrument for taking a biopsy with a rotatable coil that protrudes from a hollow needle.

US 2020/0015852 A1 discloses a needle for taking a biopsy that is provided with an expandable element to protect the sharp tip of the needle.

Both the disclosed biopsy instrument and the needle are unsuitable for generating a greater and solid tissue sample volume.

The purpose of the present invention is to provide a solution to at least one of said and other disadvantages by providing a device which allows the size of the tissue sample to be increased for one and the same diameter of the tissue receiving element.

To this end, the invention relates to an instrument for taking a tissue sample, said instrument comprising a tissue receiving element with a distal end and a proximal end and at least partly consisting of a spiral or helix, said spiral or helix comprising the distal end and having an outer surface and an inner surface and a central longitudinal axis, whereby the spiral or helix at least has a first zone whereby for each point of a first intersecting line of the outer surface and a plane of which the central longitudinal axis forms part, the distance from the outer surface to the central longitudinal axis is less than or equal to the distance from the outer surface to the central longitudinal axis on every more proximally located point along said first intersecting line, whereby the spiral or helix at least has a second zone whereby for each point of a second intersecting line of the inner surface and a plane of which the central longitudinal axis forms part, the distance from the inner surface to the central longitudinal axis (X-X') is greater than or equal to the distance from the inner surface to the central longitudinal axis on every more proximally located point along said second intersecting line.

Preferably, the first and the second zone fully coincide and preferably comprise the distal end of the tissue receiving element.

"Comprising the distal end " is understood to mean here that the distal end is part of the spiral or helix, or of the first or second zone.

Preferably, the spiral or helix of the tissue receiving element will taper off toward the distal end at its outer surface, and get wider toward the distal end at its inner surface. This tapering and widening at the outer surface and the inner surface may occur independently from each other.

This provides the advantage that the internal volume of the tissue receiving element is greater compared to the known instruments, such that a greater tissue sample volume can be taken.

Moreover, as a result of this the thickness of the spiral or helix, i.e. the distance between the inner surface and the outer surface, will decrease toward the distal end.

Another advantage is that the section of the spiral or helix at the distal end will be more flexible.

When the tissue receiving element is turned in the tissue, the distal end, or the tip of the spiral or helix, upon penetration of the spiral or helix, will tend to expand, such that a greater circle is described by the tip.

While turning the tissue receiving element, the spiral or helix will thus expand. In other words: the volume of the tissue sample will be greater than the volume enclosed by the spiral or helix before it is turned in the tissue.

Another advantage is that the spiral or helix will be thicker, and therefore less flexible at the proximal end, such that the spiral or helix has a greater stability.

It is exactly said zone at the proximal end that is exposed to the greatest torsional forces during use.

In a practical embodiment the instrument also comprises a tubular cutting element that has a distal end with a cutting edge and that fits around the tissue receiving element.

Said cutting edge can assume different forms, such as flat, tooth-shaped, etc.

The tubular cutting element will cut loose the tissue sample from the surrounding tissue by sliding the cutting element to the distal end and turning it over the tissue receiving element preferably clockwise.

The flexibility of the spiral or helix will facilitate sliding the tubular cutting element over the tissue receiving element, even when said spiral is expanded.

Consequently the tissue is lightly compressed in the tissue receiving element, such that said tissue is fixed better in the tissue receiving element. This prevents the risk of loss of tissue upon withdrawing the instrument from the tissue.

According to a preferred characteristic of the invention the inner surface and the outer surface of the spiral or helix are connected to each other by means of lateral surfaces, whereby the intersecting lines of the lateral surfaces with a plane of which the central longitudinal axis forms part, enclose an angle with the central longitudinal axis that is less than 90°, said angle pointing in proximal direction.

As a result of this, said lateral surfaces will be at an angle with the central longitudinal axis, such that a kind of barb effect is created, whereby the lateral surfaces becomes barbs as it were.

Consequently, the cross-section of the spiral or helix with a plane of which the central longitudinal axis forms part, will not be rectangular, but a parallelogram or trapezoid with an acute angle in proximal direction.

This barb effect will ensure that the tissue sample is fixedly hooked when cutting and when withdrawing the instrument from the tissue.

This hooking effect will be greater the more said angle deviates from 90°.

As a result of this, the cut off tissue sample will slide off the tissue receiving element less easily when cutting with the tubular cutting element and withdrawing the tissue receiving element from the tissue.

With the intention of better showing the characteristics of the invention a few preferred embodiments of an instrument for taking a tissue sample according to the invention are described hereinafter, by way of an example without any limiting nature, with reference to the accompanying drawings, wherein:
figure 1 schematically shows a perspective view of an instrument for taking a tissue sample according to the invention;
figure 2 schematically shows the tissue receiving element of figure 1;
figure 3 shows a cross-section of figure 2;
figure 4 shows the section indicated in figure 3 with F4 on a larger scale.

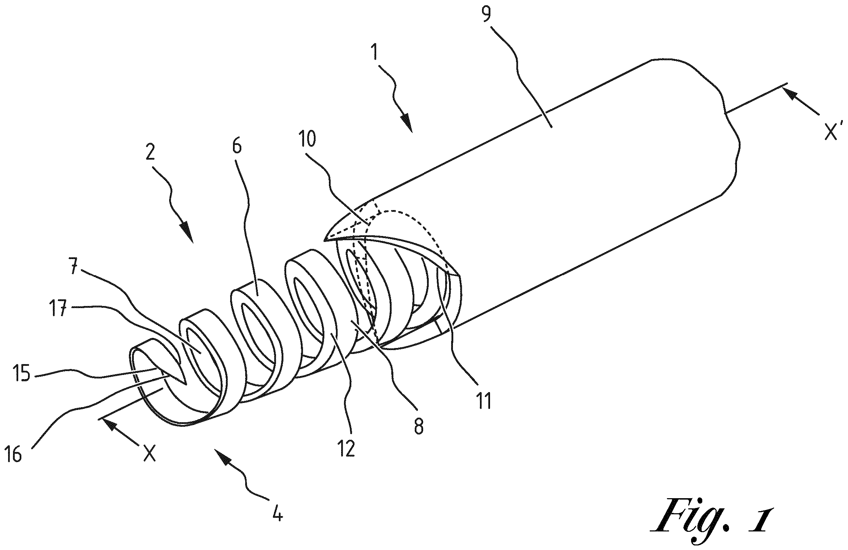

The instrument 1 for taking a tissue sample schematically shown in figure 1 comprises a tissue receiving element 2.

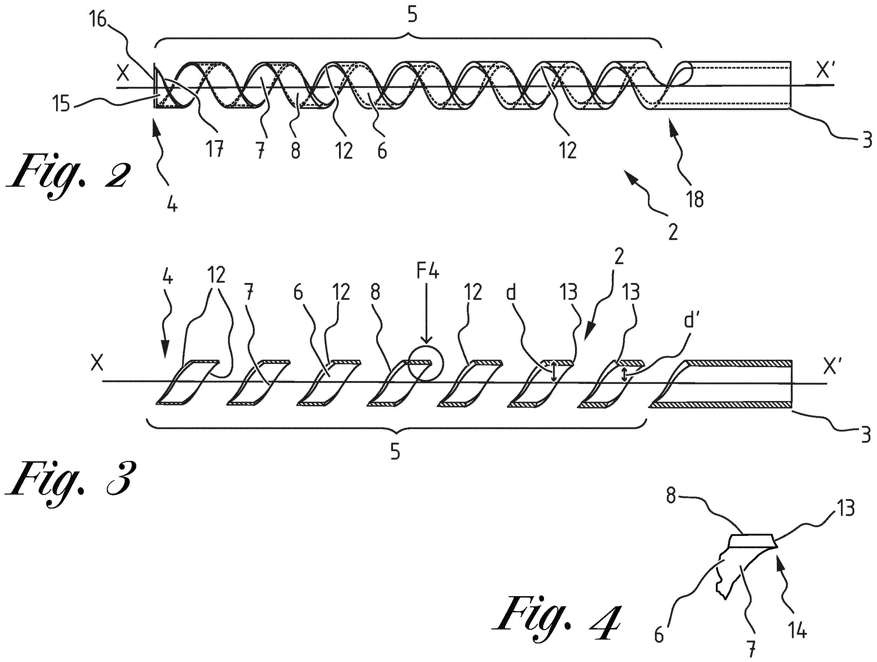

The tissue receiving element 2 is shown in detail in figure 2.

The tissue receiving element 2 has a proximal end 3 and a distal end 4.

A section 5 of the tissue receiving element 2 consists of a spiral or helix 6. The spiral or helix 6 is situated at the distal end 4, whereas the proximal end 3 is tubular.

The spiral or helix 6 comprises an inner surface 7 and an outer surface 8 and a central longitudinal axis X-X'.

The central longitudinal axis X-X' of the helix or spiral 6 coincides with the central longitudinal axis X-X' of the proximal tubular proximal end 3 of the tissue receiving element 2.

The spiral or helix 6 comprises a first zone 5 in which the spiral or helix 6 conically tapers off toward the distal end 4 at its outer surface 8.

Or in other words: at least for said first zone 5 of the spiral or helix 6, for each point of a first intersecting line of the outer surface 8 and a plane of which the central longitudinal axis (X-X') forms part, the distance d from the outer surface 8 to the central longitudinal axis X-X' will be less than or equal to the distance d from the outer surface 8 to the central longitudinal axis X-X' on every more proximally located point along said first intersecting line.

According to the invention, the spiral or helix 6 will comprise a second zone 5 in which the spiral or helix 6 conically widens toward the distal end 4 at its inner surface 7.

Or in other words: at least for said first zone 5 of the spiral or helix 6, for each point of a second intersecting line of the outer surface 7 and a plane of which the central longitudinal axis (X-X') forms part, the distance d' from the inner surface 7 to the central longitudinal axis X-X' will be greater or equal to the distance d' from the inner surface 7 to the central longitudinal axis X-X' on every more proximally located point along said second intersecting line.

In the example of figure 2 the first zone 5 and the second zone 5 fully coincide.

Moreover, the first zone 5 and the second zone 5 comprise the distal end 4.

A result of this is that the thickness of the spiral or helix 6, or in other words the distance d-d' between the inner surface 7 and the outer surface 9, can gradually decrease toward the distal end 4 of the spiral or helix 6.

The cross-section in figure 3 clearly shows the decrease of the thickness of the spiral or helix 6 toward the distal end 4.

Said decrease of the thickness ensures a certain flexibility of the spiral or helix 6, resulting in the aforementioned advantages.

Particularly the fact that as a result of this, the spiral or helix 6 will expand when turning into the tissue receiving element 2. In other words: the volume of the tissue sample will be greater than the volume enclosed by the spiral or helix 6 before it is turned into the tissue.

Furthermore, due to the internal conical widening of the spiral or helix 6 toward the distal end 4, the internal volume of the spiral or helix 6, i.e. the tubular/conical volume enclosing the spiral or helix 6, will be greater than in the known instruments for taking a tissue sample, for example tru-cut.

Next to the tissue receiving element 2, the instrument, according to the invention, also comprises in this case a tubular cutting element 9 that fits around the tissue receiving element 2.

Or in other words: the tissue receiving element 2 is fittingly enclosed in the tubular cutting element 9.

Figure 1 schematically shows an example of the cutting element 9.

In this case the cutting element 9 will fit closely rotatively and slideably lengthways around the tissue receiving element 2.

The cutting element 9 has a cutting edge 11 at its distal end 10.

The shape and size of the cutting element 9 and the tissue receiving element 2 are such that when used they cooperatively exert a cutting effect on tissue surrounding the spiral or helix 6.

The flexibility of the spiral or helix 6 will facilitate sliding the tubular cutting element 9 over the tissue receiving element 2, even when said spiral is expanded, resulting in a slight compression of the tissue sample. The resilience of the distal end 4 of the spiral or helix 6 also ensures the necessary contact between the sharp outer edge of the spiral or helix 6 and the cutting element 9.

The cross-section in figure 3 shows that the inner surface 7 and the outer surface 8 of the spiral or helix are connected with each other by means of lateral surfaces 12.

The cross-section in figure 3 is made according to a plane of which the central longitudinal axis (X-X') forms part.

The intersecting lines 13 of the lateral surfaces 12 with a plane of which the central longitudinal axis (X-X') forms part, are therefore visible in figure 3.

Said intersecting lines 13 enclose an angle with the central longitudinal axis X-X' that is less than 90°, said angle pointing in proximal direction.

Figure 4 shows a detail, which shows that in this way a kind of barb 14 is created as it were.

However, said barb 14 does not extend outside the outer surface 8 of the spiral or helix 6, and thus cannot obstruct the tubular cutting element 9, nor does it extend past the inner surface 7 of the spiral or helix 6, and thus cannot damage the tissue sample.

The barb 14 will only engage on the intersecting line made in the tissue, to anchor in the tissue sample there.

The spiral or helix 6 is provided with a tip 15 at the distal end 4 of the tissue receiving element 2.

To this end, the distal end 4 of the spiral or helix 2 is cut off according to a plane perpendicular to the central longitudinal axis X-X', such that the tip 15 is formed as a right-angled triangle, one of the right-angled sides 16 of which is formed by said cut off.

The thus created hypotenuse 17 of the tip 15, which is formed by the side of the spiral or helix 2 causes the tip 15 to lean over more towards the distal end when loading and turning the instrument 1 in the tissue, such that the helix 2 immediately hooks into the tissue. This facilitates the biopsy procedure in cavities

Due to the decrease of the thickness of the spiral or helix 6 toward the distal end 4, said tip will be very thin, which is advantageous for the production of the tissue receiving element 2.

Because there is little material at the distal end 4 of the helix or spiral 6, upon cutting off the tip 15 during production there will be little to no overheating, and as consequence of this no burr formation either.

Burr formation may not only make the tip 15 less sharp but also fragile, with a risk of breaking off when taking a biopsy.

This risk can thus be completely avoided here.

At the proximal end 18 of the spiral or helix 6, the spiral or helix 6 is attached or connected to the tubular proximal end 3 of the tissue receiving element 2.

In the transition from the spiral or helix 6 to the tubular proximal end 3, the spiral or helix 6 has a greater radius such that the attachment of the spiral or helix 6 on the tubular proximal end 3 of the tissue receiving element 2 is widened.

Due to this configuration the attachment of the spiral or helix 6 to the tubular proximal end 3 of the tissue receiving element 2 is stronger, such that the rotational or tensile force n on the helix 6 can become greater without the risk of tearing or breaking.

The operation of the instrument according to the invention is very simple and as follows.

First a localisation needle or catheter, at least composed of a so-called trocar or wire and the cutting element 9, is inserted in the location where a tissue sample needs to be taken. The positioning is done using medical imaging either radiologically or endoscopically. Once in the right spot, the helix 6 can be positioned to be turned into the tissue.

To this end, the tissue receiving element 2 is inserted through the cutting element 9. A rotational movement now turns this tissue receiving element 2 in the tissue from which a sample must be taken. The tissue in the tissue receiving element 2 is not disturbed hereby, due to the fibres running from the space within the tissue receiving element 2 to the surroundings.

As described above, the spiral or helix 6 will slightly expand, such that a greater volume of tissue is enclosed by the tissue receiving element 2.

Subsequently the cutting element 9 is moved in a distal direction, while making a rotational motion simultaneously. Jointly thanks to the interaction between the tissue receiving element 2 and the cutting element 9, tissue around the tissue receiving element 2 is cut loose.

The expansion of the spiral or helix 6 causes the cutting element 9 to exert a slight compression on the spiral or helix 6, and consequently on the tissue sample.

Subsequently, a pulling power is exerted on the instrument 1 in a proximal direction, i.e. in the direction away from the tissue. Consequently, the tissue sample tears off near the distal end 4 of the tissue receiving element 2, and it can be brought outside the patient's body for the required analyses.

Said barb effect and slight compression that the cutting element 9 exerts is important here, such that a significant force can be exerted on the tissue sample via the tissue receiving element 2, resulting in the tissue sample tearing off, and with little risk of sample loss when the whole tissue receiving element 2 is withdrawn from the tissue.

The present invention is by no means limited to the embodiments described as an example and shown in the drawings, but an instrument for taking a tissue sample according to the invention can be realised in all kinds of forms and dimensions within the scope of the invention which is defined by the appended claims.

## Claims

1. Instrument for taking a tissue sample comprising a tissue receiving element (2) that has a distal end (4) and a proximal end (3) and at least partly consists of a spiral or helix (6), said spiral or helix (6) comprising the distal end (4) and having an outer surface (8) and an inner surface (7) and a central longitudinal axis (X-X'), whereby the spiral or helix (6) at least has a first zone (5) whereby for each point of a first intersecting line of the outer surface (8) and a plane of which the central longitudinal axis (X-X') forms part, the distance (d) from the outer surface (8) to the central longitudinal axis (X-X') is less than or equal to the distance (d) of the outer surface (8) to the central longitudinal axis (X-X') on every more proximally located point along said first intersecting line, and a tubular cutting element (9) is fittingly applied around the tissue receiving element (2) and at a distal end (10) is provided with a cutting edge (11), **characterised in that** the spiral or helix (6) at least has a second zone (5) whereby for each point of a second intersecting line of the inner surface (7) and a plane of which the central longitudinal axis (X-X') forms part, the distance (d') from the inner surface (7) to the central longitudinal axis (X-X') is greater than or equal to the distance (d') from the inner surface (7) to the central longitudinal axis (X-X') on every more proximally located point along said second intersecting line, and whereby the first and the second zone (5) fully coincide and comprise the distal end (4) of the spiral or helix.

2. Instrument according to claim 1, **characterised in that** the inner surface (7) and the outer surface (8) of the spiral or helix are connected to each other by means of lateral surfaces (12), whereby the intersecting lines (13) of the lateral surfaces (12) with a plane of which the central longitudinal axis (X-X') forms part, enclose an angle with the central longitudinal axis (X-X') that is less than 90°, said angle pointing in proximal direction.

3. Instrument according to any one of the previous claims, **characterised in that** the distal end (4) of the spiral or helix (6) is cut off according to a plane perpendicular to the central longitudinal axis (X-X'), such that the distal end (4) of the spiral or helix (6) is formed as a right-angled triangle of which one of the right-angled sides (16) is formed by said cut off.

4. Instrument according to any one of the previous claims, **characterised in that** the most proximal end (3) of the tissue receiving element (2) is tubular to which said spiral or helix (6) is connected, whereby in the transition from the spiral or helix (6) to the tubular proximal end (3), the spiral or helix (6) has a greater radius such that the attachment of the spiral or helix (6) on the tubular proximal end (3) of the tissue receiving element (2) is widened.

5. Instrument according to claim 1, **characterised in that** the cutting element (9) fits closely rotatively and slideably lengthways around the tissue receiving element (2).

6. Instrument according to claim 1 or 5, **characterised in that** the shape and size of the cutting element (9) and the tissue receiving element (2) are such that when used they cooperatively exert a cutting effect on tissue surrounding the spiral or helix (6).

## Patentansprüche

1. Instrument zur Entnahme einer Gewebeprobe, das Folgendes umfasst
ein Gewebeaufnahmeelement (2), das ein distales Ende (4) und ein proximales Ende (3) besitzt und das zumindest teilweise aus einer Spirale oder Helix (6) besteht, wobei die Spirale oder Helix (6) das distale Ende (5) umfasst und eine äußere Oberfläche (8) und eine innere Oberfläche (7) sowie eine zentrale Längsachse (X-X') besitzt; wobei die Spirale oder Helix (6) mindestens einen ersten Bereich (5) besitzt; wobei für jeden Punkt einer ersten Schnittlinie der äußeren Oberfläche (8) und einer Ebene, von der die zentrale Längsachse (X-X') ein Teil ist, der Abstand (d), der sich von der äußeren Oberfläche (8) zur Mittellängsachse (X-X') erstreckt, kleiner oder gleich dem Abstand (d) ist, der sich von der äußeren Oberfläche (8) zur Mittellängsachse (X-X') an jedem Punkt erstreckt, der in der proximalen Richtung entlang der ersten Schnittlinie näher liegt, und
ein rohrförmiges Schneidelement (9), das eng anliegend um das Gewebeaufnahmeelement (2) herum angebracht ist und das an einem distalen Ende (10) mit einer Schneidkante (11) versehen ist; **dadurch gekennzeichnet, dass** die Spirale oder Helix (6) mindestens einen zweiten Bereich (5) besitzt; wobei für jeden Punkt einer zweiten Schnittlinie der inneren Oberfläche (7) und einer Ebene, von der die zentrale Längsachse (X-X') ein Teil ist, der Abstand (d'), der sich von der inneren Oberfläche (7) zur zentralen Längsachse (X-X') bis zur zentralen Längsachse (X-X') erstreckt, größer oder gleich dem Abstand (d') ist, der sich von der inneren Oberfläche (7) zur zentralen Längsachse (X-X') erstreckt auf jedem Punkt, der in der proximalen Richtung entlang der zweiten Schnittlinie näher liegt und wobei der erste und der zweite Bereich (5) vollständig zusammenfallen und das distale Ende (4) der Spirale oder Helix umfassen.

2. Instrument nach Anspruch 1, **dadurch gekennzeichnet, dass** die innere Oberfläche (7) und die äußere Oberfläche (8) der Spirale oder Helix mittels seitlichen Oberflächen (12) miteinander verbunden sind; wobei die Schnittlinien (13) der seitlichen Oberflächen (12) mit einer Ebene, von der die zentrale Längsachse (X-X') ein Teil ist, einen Winkel mit der zentralen Längsachse (X-X') bilden, der kleiner als 90° ist, wobei der oben genannte Winkel in die proximale Richtung zeigt.

3. Instrument nach einem der vorhergehenden Ansprüche, **dadurch gekennzeichnet, dass** das distale Ende (4) der Spirale oder Helix (6) gemäß einer Ebene, die senkrecht zur zentralen Längsachse (X-X') ist, in einer solchen Weise ausgeschnitten ist, dass das distale Ende (4) der Spirale oder Helix (6) in der Form eines rechtwinkligen Dreiecks gebildet wird, von dem eine der rechtwinkligen Seiten (16) durch den Schnitt gebildet wird.

4. Instrument nach einem der vorhergehenden Ansprüche, **dadurch gekennzeichnet, dass** das proximalste Ende (3) des Gewebeaufnahmeelements (2) eine röhrenförmige Form bildet, mit der die Spirale oder Helix (6) verbunden ist; wobei in dem Übergang zwischen der Spirale oder Helix (6) und dem rohrförmigen proximalen Ende (3) die Spirale oder Helix (6) einen größeren Radius in einer Weise besitzt, dass eine Aufweitung der Befestigung der Spirale oder Helix (6) an dem rohrförmigen proximalen Ende (3) des Gewebeaufnahmeelements (2) erzielt wird.

5. Instrument nach Anspruch 1, **dadurch gekennzeichnet, dass** das Schneideelement (9) in der Drehung und in der seitlichen Position über die Länge des Gewebeaufnahmeelements (2) perfekt passt.

6. Instrument nach Anspruch 1 oder 5, **dadurch gekennzeichnet, dass** die Konfiguration und die Größe des Schneidelements (9) und des Gewebeaufnahmeelements (2) so beschaffen sind, dass sie während der Verwendung der letztgenannten Elemente zusammenwirken, um eine Schneidwirkung auf das Gewebe auszuüben, der die Spirale oder Helix (6) umgibt.

## Revendications

1. Instrument destiné à prélever un échantillon de tissu, qui comprend
un élément de réception de tissu (2) qui possède une extrémité distale (4) et une extrémité proximale (3) et qui est constitué au moins en partie d'une spirale ou d'une hélice (6), dans lequel ladite spirale au ladite hélice (6) comprend l'extrémité distale (5) et possède une surface externe (8) et une surface interne (7), ainsi qu'un axe longitudinal central (X-X') ; dans lequel la spirale ou l'hélice (6) possède au moins une première zone (5) ; dans lequel, pour chaque point d'une première ligne d'intersection de la surface externe (8) et d'un plan dont fait partie l'axe longitudinal central (X-X'), la distance (d) qui s'étend à partir de la surface externe (8) jusqu'à l'axe longitudinal central (X-X') est inférieure ou égale à la distance (d) qui s'étend à partir de la surface externe (8) jusqu'à l'axe longitudinal central (X-X') sur chaque point qui est situé plus près dans la direction proximale le long de ladite première ligne d'intersection, et
un élément tubulaire de coupe (9) qui est appliqué en ajustage serré autour de l'élément de réception de tissu (2), et qui est muni, à une extrémité distale (10), d'un bord coupant (11) ; **caractérisé en ce que** la spirale ou l'hélice (6) possède au moins une deuxième zone (5) ; dans lequel, pour chaque point d'une deuxième ligne d'intersection de la surface interne (7) et d'un plan dont fait partie l'axe longitudinal central (X-X'), la distance (d') qui s'étend à partir de la surface interne (7) jusqu'à l'axe longitudinal central (X-X') jusqu'à l'axe longitudinal central (X-X') est supérieure ou égale à la distance (d') qui s'étend à partir de la surface interne (7) jusqu'à l'axe longitudinal central (X-X') sur chaque point qui est situé plus près dans la direction proximale le long de ladite deuxième ligne d'intersection ; et dans lequel la première et la deuxième zone (5) coïncident totalement et comprennent l'extrémité distale (4) de la spirale ou de l'hélice.

2. Instrument selon la revendication 1, **caractérisé en ce que** la surface interne (7) et la surface externe (8) de la spirale ou de l'hélice sont reliées l'une à l'autre au moyen de surfaces latérales (12) ; dans lequel les lignes d'intersection (13) des surfaces latérales (12) avec un plan dont fait partie l'axe longitudinal central (X-X'), forment un angle avec l'axe longitudinal central (X-X') qui est inférieur à 90°, l'angle en question pointant dans la direction proximale.

3. Instrument selon l'une quelconque des revendications précédentes, **caractérisé en ce que** l'extrémité distale (4) de la spirale ou de l'hélice (6) est découpée conformément à un plan qui est perpendiculaire à l'axe longitudinal central (X-X'), d'une manière telle que l'extrémité distale (4) de la spirale ou de l'hélice (6) est réalisée sous la forme d'un triangle rectangle dont un des côtés à angle droit (16) est réalisé par l'intermédiaire de ladite découpe.

4. Instrument selon l'une quelconque des revendications précédentes, **caractérisé en ce que** l'extrémité la plus proximale (3) de l'élément de réception de tissu (2) présente une forme tubulaire à laquelle est reliée ladite spirale ou ladite hélice (6) ; dans lequel dans la transition entre la spirale ou l'hélice (6) et l'extrémité proximale tubulaire (3), la spirale ou l'hélice (6) possède un rayon supérieur d'une manière telle que l'on obtient un élargissement de la fixation de la spirale ou de l'hélice (6) sur l'extrémité proximale tubulaire (3) de l'élément de réception de tissu (2).

5. Instrument selon la revendication 1, **caractérisé en ce que** l'élément de coupe (9) s'adapte parfaitement en rotation et en position latérale sur la longueur de l'élément de réception de tissu (2).

6. Instrument selon la revendication 1 ou 5, **caractérisé en ce que** la configuration et la dimension de l'élément de coupe (9) et de l'élément de réception de tissu (2) sont telles que, au cours de l'utilisation des derniers cités, ceux-ci coopèrent pour exercer un effet de coupe sur le tissu qui entoure la spirale ou l'hélice (6).
